# EUROPÄISCHE PATENTSCHRIFT

(11) **EP 2 869 898 B1**
(45) Veröffentlichungstag und Bekanntmachung des Hinweises auf die Patenterteilung: **07.08.2019**
(21) Anmeldenummer: 13732500.7
(22) Anmeldetag: 28.06.2013
(51) Int. Cl.: A61Q 5/06, A61K 8/25, A61K 8/58, A61K 8/34, A61K 8/42, A61K 8/44, A61K 8/46

(54) **MITTEL UND VERFAHREN ZUR TEMPORÄREN VERFORMUNG KERATINHALTIGER FASERN**
AGENT AND METHOD FOR THE TEMPORARY DEFORMATION OF KERATIN FIBRES
PRODUITS ET PROCÉDÉS POUR METTRE EN FORME TEMPORAIREMENT DES FIBRES KÉRATINIQUES

(30) Priorität: 09.07.2012 DE 102012211912
(43) Veröffentlichungstag der Anmeldung: 13.05.2015
(73) Patentinhaber: Henkel AG & Co. KGaA, 40589 Düsseldorf (DE)
(72) Erfinder: KNAPPE, Thorsten, 22869 Schenefeld (DE); HENSCHEL, Anna, 21423 Winsen (Luhe) (DE)
(86) Internationale Anmeldenummer: PCT/EP2013/063592
(87) Internationale Veröffentlichungsnummer: WO 2014/009174

(56) Entgegenhaltungen:
- WO-A1-2011/076518
- WO-A1-2012/168073
- US-A1- 2006 246 027
- Evonik Industries: "Newsletter for silica in personal care Issue 2 | 2012", , 19. April 2012 (2012-04-19), XP002726946, Gefunden im Internet: URL:http://www.sipernat.com/product/sipern at/Documents/Beauty-Bulletin-2012-02-EN.pd f [gefunden am 2014-07-10]
- HASENZAHL S ET AL: "Fumed silica for personal care and cosmetics-versatile and effective", SOFW-JOURNAL SEIFEN, OELE, FETTE, WACHSE, VERLAG FUR CHEMISCHE INDUSTRIE, AUGSBURG, DE, Bd. 129, Nr. 8, 1. Januar 2003 (2003-01-01), Seite 22, XP001539786, ISSN: 0942-7694

## Beschreibung

Die Anmeldung betrifft das technische Gebiet der temporären Verformung keratinhaltiger Fasern, insbesondere menschlicher Haare.

Stylingmittel zur Verformung keratinhaltiger Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.
Entsprechende Mittel zur temporären Formgebung enthalten als formgebende Komponente üblicherweise synthetische Polymere. Zubereitungen, die ein Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Bekannte Formen temporärer Stylingmittel lassen sich oftmals nicht mit zufrieden stellender Genauigkeit dosieren. So lassen sich etwa Haargele, Haarcremes und Haarwachse nur noch schwierig verteilen, sobald sie einmal auf das Haar aufgebracht sind.
Haarsprays lassen sich gleichmäßiger auf das Haar verteilen. Da der Verwender aber keine Möglichkeit hat, die Gesamtmenge an aufgebrachtem Stylingmittel visuell zu erfassen, besteht die Gefahr, dass mehr Stylingmittel auf das Haar aufgebracht wird, als eigentlich erforderlich wäre.

Pulverförmige Kosmetika sind bekannt und werden etwa im Bereich der Hautbehandlung bereits seit langem eingesetzt. Typische Beispiele sind etwa Make-up Puder oder Lidschatten. Um die pulverförmige Konsistenz zu erzielen, ist der Einsatz eines pulverförmigen Trägermaterials erforderlich. Als geeignetes Trägermaterial kann etwa ein Metalloxid, wie z.B. Siliziumdioxid verwendet werden. Besonders interessant ist hydrophobiertes Metalloxid bzw. Siliziumdioxid. Dieses kann beispielsweise ausgehend von pyrogenem Siliziumdioxid, das in verschiedenen Spezifikationen kommerziell erhältlich ist, erhalten werden. Auf der Oberfläche trägt unbehandeltes pyrogenes Siliziumdioxid Silanol- und Siloxangruppen. Dadurch hat es eine hohe Affinität zu Wasser, d.h. es ist hydrophil. Durch Umsetzung mit geeigneten organischen Siliziumverbindungen lassen sich Alkylsilylgruppen auf der Oberfläche des pyrogenen Siliziumdioxids chemisch binden. Es entstehen modifizierte Siliziumdioxidpulver, die nicht mehr von Wasser benetzt werden, d.h. die hydrophobe Eigenschaften aufweisen. Diese hydrophobierten Siliciumdioxied eignen sich zur Herstellung von so genanntem trockenem Wasser, bei welchem die Wassertropfen am erneuten Zusammenfließen gehindert werden. Die resultierenden pulverförmigen Feststoffe können Wassergehalt von bis zu 95% aufweisen. Unter mechanischer Beanspruchung, beispielsweise beim Verreiben auf der Haut, wird das eingeschlossene Wasser wieder freigesetzt.

Kosmetische oder pharmazeutische, verflüssigbare Pulverzusammensetzungen werden beispielsweise in dem europäischen Patent EP 1 235 554 B1 beschrieben.
Die internationale Anmeldung WO 03/037287 A1 offenbart die Verwendung eines Granulats auf Basis von pyrogenem Siliziumdioxid in kosmetischen Zusammensetzungen. Die speziellen Granulate können silanisiert, d.h. hydrophobiert werden und eignen sich zur Herstellung kosmetischer Zusammensetzungen jeglicher Konsistenz, beispielsweise Flüssigkeiten, Schäume, Sprays oder Pulver.
Die internationale Anmeldung WO 2007/051511 A1 beschreibt die Verwendung einer pulverförmigen Zusammensetzung, enthaltend 50 bis 95 Gew.-% eines wässrigen Lösungsmittels, hydrophobiertes Siliziumdioxidpulver und mindestens im wässrigen Lösemittel befindliches filmbildendes und/oder festigendes Polymer zur temporären Verformung keratinischer Fasern.
Die deutsche Patentanmeldung DE102008057261 A1 hat pulverförmige Zusammensetzungen zum Gegenstand, die zur temporären Umformung von Haaren für sehr starkem Halt der fixierten Frisur eingesetzt werden.

Die pulverförmigen Haarkosmetika des Standes der Technik liefen mittlerweile zwar einen für die Haarumformung akzeptablen Halt und zeichnen sich durch eine gute Dosierbarkeit aus. Allerdings ist das mit diesen Mitteln erzielte Ergebnis bezüglich der Parameter natürlicher Glanz sowie der Elastizität und des Halts verbesserungswürdig. Zudem sind die meisten üblichen Stylingrohstoffe wie Wachse, Öle oder Polymere nicht ohne weiteres zur Herstellung stabiler pulverförmiger Zusammensetzungen geeignet. Entweder verhindern sie die erfolgreiche Bildung der Kern-Hülle-Teilchen, oder die Lagerstabilität der gebildeten Kern-Hülle-Teilchen wird herabgesenkt.

Aufgabe der vorliegenden Erfindung war es daher, lagerstabile pulverförmige Haarbehandlungsmittel zur temporären Formgebung zur Verfügung zu stellen, die sich genau und einfach dosiert lassen, das Haar nicht verkleben, dem Haar einen volleren sowie natürlichen Griff und natürlichen Glanz verleihen. Die Haltbarkeit des Stylingergebnisses soll dabei nicht beeinträchtigt werden.

Es wurde festgestellt, dass pulverförmige kosmetische Zusammensetzungen auf Grundlage eines Polyol-Emulgator Gemisches die vorgenannten Aufgabe lösen. Ein erster Gegenstand der vorliegenden Anmeldung sind pulverförmige kosmetische Zusammensetzungen, enthaltend
a) 5 bis 20 Gew.-% hydrophobiertes Metalloxidpulver, enthaltend hydrophobiertes Silikat,
b) 40 bis 94 Gew.-% organisches Polyol, enthaltend mindestens eine Verbindung aus der Gruppe Glycerin, Sorbitol und Panthenol,
c) 0,1 bis 15 Gew.-% Emulgator, enthaltend einen bei Raumtemperatur (20°C) festen anionischen Emulgator,
d) 0 bis 40 Gew.-% Wasser.

Die erfindungsgemäßen pulverförmigen Zusammensetzungen liegen vorzugsweise in Form von Kern-Hülle-Teilchen vor, deren Hülle Partikel mindestens eines hydrophobierten Metalloxidpulvers sowie den Emulgator enthält und deren flüssiger Kern organisches Polyol sowie gegebenenfalls Wasser enthält.
Partikel sind im Sinne der Erfindung als Korn vorliegende Teilchen (vgl. DIN 66160: 1992-09) von Festkörpern.
Pulverförmig im Sinne der Erfindung sind Zusammensetzungen, deren Teilchen unter eigenem Gewicht frei rieselfähig sind (vgl. DIN EN ISO 6186: 1998-08).

Die erfindungsgemäßen, pulverförmigen Zusammensetzungen zeichnen sich dadurch aus, dass der flüssige Kern durch mechanische Belastung der Kern-Hülle-Teilchen, insbesondere durch Reibung und/oder Druck, aus dem Kern-Hülle-Teilchen freigesetzt wird und sich dabei aus der pulverförmigen Zusammensetzung eine Flüssigkeit bildet. Es handelt sich somit um eine pulverförmige powder-to-liquid Zusammensetzung. Die erfindungsgemäßen pulverförmigen Zusammensetzungen können sehr einfach dosiert werden. Sie lassen sich zudem sehr gleichmäßig im Haar verteilen, da der flüssige Kern erst unter mechanischer Beanspruchung am Wirkort freigesetzt und eine gezielte Benetzung der Haarfasern ermöglicht wird. Das Pulver kann also zunächst vorsichtig im Haar verteilt und erst anschließend stärker mechanisch belastet werden, beispielsweise durch gezieltes Einmassieren des Pulvers in das Haar. Dadurch wird der Stylingeffekt erst direkt auf der gewünschten Haarpartie entfaltet.

Die verwendeten pulverförmigen Zusammensetzungen enthalten hydrophobiertes Metalloxid. Bevorzugte Zusammensetzungen sind dadurch gekennzeichnet, dass sie das hydrophobierte Metalloxidpulver bezogen auf ihr Gesamtgewicht in Mengen von 5,5 bis 18, vorzugsweise von 6 bis 15 Gew.-% enthalten. Die optimale Menge hängt dabei vor allem von der Hydrophobizität des eingesetzten Siliziumdioxidpulvers ab. Je hydrophober das Siliziumdioxidpulver ist, desto weniger davon wird benötigt, um ein stabiles, pulverförmiges Produkt zu erhalten.

Die Art des hydrophobierten Metalloxids ist nicht prinzipiell beschränkt, solange sichergestellt ist, dass beim intensiven Vermischen mit der flüssigen, wässrigen Phase ein pulverförmiges Produkt entsteht. Unter hydrophobiert sind im Sinne der Erfindung solche Metalloxide zu verstehen, die zumindest an der Oberfläche der Partikel derart modifiziert wurden, dass das modifizierte Teilchen weniger von Wasser benetzt wird als das nicht modifizierte Teilchen. Insbesondere sind silanisierte, hydrophobierte Metalloxide bevorzugt. Als Reagenz zur Silanisierung des Metalloxids eignet sich erfindungsgemäß bevorzugt mindestens ein Vertreter der Gruppe, die gebildet wird, aus Silanen, Halogensilanen, Alkoxysilanen und Silazanen. Bevorzugt geeignete hydrophobierte Metalloxide des hydrophobierten Metalloxidpulvers werden erfindungsgemäß ausgewählt aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid. Für die Herstellung der erfindungsgemäßen kosmetischen Zusammensetzungen besonders geeignet haben sich mit Polydimethylsiloxan nachbehandelte pyrogene Kieselsäure. Entsprechende Metalloxide mit der INCI Bezeichung "Silica Dimethicone Silylate" werden beispielsweise von der Firma Evonik unter dem Handelnnamen Aersoil® R202 vertrieben.

Der Partikeldurchmesser der Primärteilchen bevorzugter hydrophobierter Metalloxide beträgt vorzugsweise weniger als 5 µm, besonders bevorzugt weniger als 1 µm, und insbesondere zwischen 1 und 50 nm.

Bevorzugt sind weiterhin solche hydrophobierte Siliziumdioxide, die eine spezifische Oberfläche nach BET zwischen 10 und 400 m²/g, vorzugsweise zwischen 40 bis 300 m²/g und insbesondere 80 bis 150 m²/g aufweisen.

Die erfindungsgemäßen pulverförmigen Zusammensetzungen enthalten als zweiten wesentlichen Bestandteil ein organisches Polyol. Bevorzugte Zusammensetzungen enthalten das organische Polyol bezogen auf ihr Gesamtgewicht in Mengen von 45 bis 92 Gew.-%, vorzugsweise von 60 bis 90 Gew.-% und insbesondere von 70 bis 88 Gew.-%. Das Polyol kann als Einzelsubstanz oder in Form von Polyol Mischungen eingesetzt werden. Bevorzugte erfindungsgemäße Mittel zeichnen sich dadurch aus, dass sie weniger als vier, vorzugsweise ein bis drei, insbesondere jedoch nur ein Polyol enthalten. Zur Herstellung kosmetischer Zusammensetzungen eignen sich dabei insbesondere Glycerin, Sorbitol und Panthenol. Diese Polyole können durch vergleichsweise geringe Mengen hydrophobierten Metalloxidpulvers, in der Regel mit weniger als 10 Gew.-% Metalloxidpulver (bezogen auf das Gesamtgewicht der pulverförmigen kosemtischen Zusammensetzung), in eine Pulverform überführt werden.

Die erfindungsgemäße Zusammensetzung enthält als organisches Polyol mindestens eine Verbindung aus der Gruppe Glycerin, Sorbitol und Panthenol.

Ein weiterer wesentlicher Bestandteil der erfindungsgemäßen Zusammensetzungen ist ein Emulgator. Bevorzugte Zusammensetzungen enthalten den Emulgator bezogen auf ihr Gesamtgewicht in Mengen von 1,0 bis 12 Gew.-%, vorzugsweise 2,0 bis 11 Gew.-% und insbesondere von 5,0 bis 10 Gew.-%.

Die kosmetischen Mittel enthalten einen bei Raumtemperatur (20°C) festen anionischen Emulgator.
Anionische Emulgatoren sind durch eine hydrophilbe anionische Gruppe, z. B. eine Carboxylat-, Sulfat-, Sulfonat- oder Phosphat-Gruppe und eine lipophile Alkylgruppe gekennzeichnet. Zusätzlich können im Molekül Glycol- oder Polyglycolether-Gruppen, Ester-, Ether- und Amidgruppen sowie Hydroxylgruppen enthalten sein. Beispiele für geeignete anionische Tenside und Emulgatoren sind (jeweils in Form der Natrium-, Kalium- und Ammonium- sowie der Mono-, Di- und Trialkanolammoniumsalze mit 2 bis 4 C-Atomen in der Alkanolgruppe):
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
   Acylglutamate, insbesondere Natrium-N-cocoyl- und Natrium-N-stearoyl-L-glutamat,
- Ester der Sulfobernsteinsäure oder der Sulfosuccinate der allgemeinen Formel (T3I), 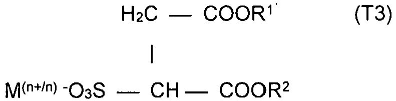 in der M^{(n+/n)} für n = 1 ein Wasserstoffatom, ein Alkalimetallkation, eine Ammoniumgruppe oder das Kation einer ammonium-organischen Base und für n = 2 ein Erdalkalimetallkation darstellt und R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt ist, unter der Maßgabe, dass wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alkylsulfate und Alkylpolyglycolethersulfate der Formel R-(O-CH₂-CH₂)ₓ-OSO₃H, in der R eine bevorzugt lineare Alkylgruppe mit 8 bis 30 C-Atomen und x = 0 oder 1 - 12 ist,
- gemischte oberflächenaktive Hydroxysulfonate gemäß DE-A-37 25 030,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate,
- sulfatierte Fettsäurealkylenglycolester,
- Monoglyceridsulfate und Monoglyceridethersulfate.
Besonders bevorzugte anionische Emulgatoren sind Cetearyl Sulfosuccinate und Stearoyl Glutamate.

Zusammenfassend erhalten bevorzugte erfindungsgemäße kosmetische Zusammensetzung als Emulgator mindestens einen Emulgator aus der Gruppe der Cetearyl Sulfosuccinate und Stearoyl Glutamate.

Die erfindungsgemäßen kosmetischen Zusammensetzungen können schließlich auch Wasser enthalten. Die Herstellung wasserarmer Zubereitungen hat sich jedoch für die kosmetischen Eigenschaften der erfindungsgemäßen Zubereitungen als vorteilhaft erwiesen. Erfindungsgemäß bevorzugte kosmeitische Zusammensetzungen sind daher dadurch gekennzeichnet, dass sie bezogen auf ihr Gesamtgewicht weniger als 25 Gew.-%, vorzugsweise weniger als 10 Gew.-%, bevorzugt weniger als 5,0 Gew.-% und insbesondere weniger als 1,0 Gew.-% Wasser enthalten.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Polyol²⁾ | 40 bis 94 | 45 bis 92 | 60 bis 90 | 70 bis 88 | 70 bis 88 |
| Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 6 | Formel 7 | Formel 8 | Formel 9 | Formel 10 |
|---|---|---|---|---|---|
| Silica Dimethicone Silylate | 5 bis 20 | 5,5 bis 18 | 6,0 bis 15 | 6,0 bis 10 | 6,0 bis 10 |
| Polyol²⁾ | 40 bis 94 | 45 bis 92 | 60 bis 90 | 70 bis 88 | 70 bis 88 |
| Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 11 | Formel12 | Formel 13 | Formel 14 | Formel 15 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Glycerin | 40 bis 94 | 45 bis 92 | 60 bis 90 | 70 bis 88 | 70 bis 88 |
| Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 16 | Formel 17 | Formel 18 | Formel 19 | Formel 20 |
|---|---|---|---|---|---|
| Silica Dimethicone Silylate | 5 bis 20 | 5,5 bis 18 | 6,0 bis 15 | 6,0 bis 10 | 6,0 bis 10 |
| Glycerin | 40 bis 94 | 45 bis 92 | 60 bis 90 | 70 bis 88 | 70 bis 88 |
| Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 21 | Formel 22 | Formel 23 | Formel 24 | Formel 25 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Sorbitol | 40 bis 94 | 45 bis 92 | 60 bis 90 | 70 bis 88 | 70 bis 88 |
| Emulgator³⁾ | 0,1 bis 15 | 1,0 bis 12 | 2,0 bis 11 | 5,0 bis 10 | 5,0 bis 10 |
| Wasser | 10 bis 40 | 10 bis 25 | 10 bis 25 | 10 bis 25 | 15 bis 25 |

| | Formel 26 | Formel 27 | Formel 28 | Formel 29 | Formel 30 |
|---|---|---|---|---|---|
| Silica Dimethicone Silylate | 5 bis 20 | 5,5 bis 18 | 6,0 bis 15 | 6,0 bis 10 | 6,0 bis 10 |
| Sorbitol | 40 bis 94 | 45 bis 92 | 60 bis 90 | 70 bis 88 | 70 bis 88 |
| Emulgator³⁾ | 0,1 bis 15 | 1,0 bis 12 | 2,0 bis 11 | 5,0 bis 10 | 5,0 bis 10 |
| Wasser | 10 bis 40 | 10 bis 25 | 10 bis 25 | 10 bis 25 | 15 bis 25 |

| | Formel 31 | Formel 32 | Formel 33 | Formel 34 | Formel 35 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Panthenol | 40 bis 94 | 45 bis 92 | 60 bis 90 | 70 bis 88 | 70 bis 88 |
| Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 36 | Formel 37 | Formel 38 | Formel 39 | Formel 40 |
|---|---|---|---|---|---|
| Silica Dimethicone Silylate | 5 bis 20 | 5,5 bis 18 | 6,0 bis 15 | 6,0 bis 10 | 6,0 bis 10 |
| Panthenol | 40 bis 94 | 45 bis 92 | 60 bis 90 | 70 bis 88 | 70 bis 88 |
| Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 41 | Formel 42 | Formel 43 | Formel 44 | Formel 45 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Polyol²⁾ | 40 bis 94 | 45 bis 92 | 60 bis 90 | 70 bis 88 | 70 bis 88 |
| anionischer Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 46 | Formel 47 | Formel 48 | Formel 49 | Formel 50 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Polyol²⁾ | 40 bis 94 | 45 bis 92 | 60 bis 90 | 70 bis 88 | 70 bis 88 |
| Cetearylsulfosuccinat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 51 | Formel 52 | Formel 53 | Formel 54 | Formel 55 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Polyol²⁾ | 40 bis 94 | 45 bis 92 | 60 bis 90 | 70 bis 88 | 70 bis 88 |
| Stearoylglutamat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 56 | Formel 57 | Formel 58 | Formel 59 | Formel 60 |
|---|---|---|---|---|---|
| Silica Dimethicone Silylate | 5 bis 20 | 5,5 bis 18 | 6,0 bis 15 | 6,0 bis 10 | 6,0 bis 10 |
| Polyol²⁾ | 40 bis 94 | 45 bis 92 | 60 bis 90 | 70 bis 88 | 70 bis 88 |
| anionischer Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 61 | Formel 62 | Formel 63 | Formel 64 | Formel 65 |
|---|---|---|---|---|---|
| Silica Dimethicone Silylate | 5 bis 20 | 5,5 bis 18 | 6,0 bis 15 | 6,0 bis 10 | 6,0 bis 10 |
| Polyol²⁾ | 40 bis 94 | 45 bis 92 | 60 bis 90 | 70 bis 88 | 70 bis 88 |
| Cetearylsulfosuccinat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 66 | Formel 67 | Formel 68 | Formel 69 | Formel 70 |
|---|---|---|---|---|---|
| Silica Dimethicone Silylate | 5 bis 20 | 5,5 bis 18 | 6,0 bis 15 | 6,0 bis 10 | 6,0 bis 10 |
| Polyol²⁾ | 40 bis 94 | 45 bis 92 | 60 bis 90 | 70 bis 88 | 70 bis 88 |
| Stearoylglutamat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 71 | Formel 72 | Formel 73 | Formel 74 | Formel 75 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Glycerin | 40 bis 94 | 45 bis 92 | 60 bis 90 | 70 bis 88 | 70 bis 88 |
| anionischer Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 76 | Formel 77 | Formel 78 | Formel 79 | Formel 80 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Sorbitol | 40 bis 94 | 45 bis 92 | 60 bis 90 | 70 bis 88 | 70 bis 88 |
| anionischer Emulgator³⁾ | 0,1 bis 15 | 1,0 bis 12 | 2,0 bis 11 | 5,0 bis 10 | 5,0 bis 10 |
| Wasser | 10 bis 40 | 10 bis 25 | 10 bis 25 | 10 bis 25 | 15 bis 25 |

| | Formel 81 | Formel 82 | Formel 83 | Formel 84 | Formel 85 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Panthenol | 40 bis 94 | 45 bis 92 | 60 bis 90 | 70 bis 88 | 70 bis 88 |
| anionischer Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 86 | Formel 87 | Formel 88 | Formel 89 | Formel 90 |
|---|---|---|---|---|---|
| Silica Dimethicone Silylate | 5 bis 20 | 5,5 bis 18 | 6,0 bis 15 | 6,0 bis 10 | 6,0 bis 10 |
| Glycerin | 40 bis 94 | 45 bis 92 | 60 bis 90 | 70 bis 88 | 70 bis 88 |
| anionischer Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 91 | Formel 92 | Formel 93 | Formel 94 | Formel 95 |
|---|---|---|---|---|---|
| Silica Dimethicone Silylate | 5 bis 20 | 5,5 bis 18 | 6,0 bis 15 | 6,0 bis 10 | 6,0 bis 10 |
| Sorbitol | 40 bis 94 | 45 bis 92 | 60 bis 90 | 70 bis 88 | 70 bis 88 |
| anionischer Emulgator³⁾ | 0,1 bis 15 | 1,0 bis 12 | 2,0 bis 11 | 5,0 bis 10 | 5,0 bis 10 |
| Wasser | 10 bis 40 | 10 bis 25 | 10 bis 25 | 10 bis 25 | 15 bis 25 |

| | Formel 96 | Formel 97 | Formel 98 | Formel 99 | Formel 100 |
|---|---|---|---|---|---|
| Silica Dimethicone Silylate | 5 bis 20 | 5,5 bis 18 | 6,0 bis 15 | 6,0 bis 10 | 6,0 bis 10 |
| Panthenol | 40 bis 94 | 45 bis 92 | 60 bis 90 | 70 bis 88 | 70 bis 88 |
| anionischer Emulgator³⁾ | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 101 | Formel 102 | Formel 103 | Formel 104 | Formel 105 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Glycerin | 40 bis 94 | 45 bis 92 | 60 bis 90 | 70 bis 88 | 70 bis 88 |
| Cetearylsulfosuccinat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 106 | Formel 107 | Formel 108 | Formel109 | Formel 110 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Sorbitol | 40 bis 94 | 45 bis 92 | 60 bis 90 | 70 bis 88 | 70 bis 88 |
| Cetearylsulfosuccinat | 0,1 bis 15 | 1,0 bis 12 | 2,0 bis 11 | 5,0 bis 10 | 5,0 bis 10 |
| Wasser | 10 bis 40 | 10 bis 25 | 10 bis 25 | 10 bis 25 | 15 bis 25 |

| | Formel 111 | Formel 112 | Formel 113 | Formel 114 | Formel 115 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Panthenol | 40 bis 94 | 45 bis 92 | 60 bis 90 | 70 bis 88 | 70 bis 88 |
| Cetearylsulfosuccinat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 116 | Formel 117 | Formel 118 | Formel 119 | Formel 120 |
|---|---|---|---|---|---|
| Silica Dimethicone Silylate | 5 bis 20 | 5,5 bis 18 | 6,0 bis 15 | 6,0 bis 10 | 6,0 bis 10 |
| Glycerin | 40 bis 94 | 45 bis 92 | 60 bis 90 | 70 bis 88 | 70 bis 88 |
| Cetearylsulfosuccinat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 121 | Formel 122 | Formel 123 | Formel 124 | Formel 125 |
|---|---|---|---|---|---|
| Silica Dimethicone Silylate | 5 bis 20 | 5,5 bis 18 | 6,0 bis 15 | 6,0 bis 10 | 6,0 bis 10 |
| Sorbitol | 40 bis 94 | 45 bis 92 | 60 bis 90 | 70 bis 88 | 70 bis 88 |
| Cetearylsulfosuccinat | 0,1 bis 15 | 1,0 bis 12 | 2,0 bis 11 | 5,0 bis 10 | 5,0 bis 10 |
| Wasser | 10 bis 40 | 10 bis 25 | 10 bis 25 | 10 bis 25 | 15 bis 25 |

| | Formel 126 | Formel 127 | Formel 128 | Formel 129 | Formel 130 |
|---|---|---|---|---|---|
| Silica Dimethicone Silylate | 5 bis 20 | 5,5 bis 18 | 6,0 bis 15 | 6,0 bis 10 | 6,0 bis 10 |
| Panthenol | 40 bis 94 | 45 bis 92 | 60 bis 90 | 70 bis 88 | 70 bis 88 |
| Cetearylsulfosuccinat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 131 | Formel 132 | Formel 133 | Formel 134 | Formel 135 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Glycerin | 40 bis 94 | 45 bis 92 | 60 bis 90 | 70 bis 88 | 70 bis 88 |
| Stearoylglutamat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 136 | Formel 137 | Formel 138 | Formel 139 | Formel 140 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Sorbitol | 40 bis 94 | 45 bis 92 | 60 bis 90 | 70 bis 88 | 70 bis 88 |
| Stearoylglutamat | 0,1 bis 15 | 1,0 bis 12 | 2,0 bis 11 | 5,0 bis 10 | 5,0 bis 10 |
| Wasser | 10 bis 40 | 10 bis 25 | 10 bis 25 | 10 bis 25 | 15 bis 25 |

| | Formel 141 | Formel 142 | Formel 143 | Formel 144 | Formel 145 |
|---|---|---|---|---|---|
| Hydrophobiertes Metalloxidpulver¹⁾ | 5 bis 20 | 5,5 bis 18 | 6,0 bis 15 | 6,0 bis 10 | 6,0 bis 10 |
| Panthenol | 40 bis 94 | 45 bis 92 | 60 bis 90 | 70 bis 88 | 70 bis 88 |
| Stearoylglutamat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 146 | Formel 147 | Formel 148 | Formel 149 | Formel 150 |
|---|---|---|---|---|---|
| Silica Dimethicone Silylate | 5 bis 20 | 5,5 bis 18 | 6,0 bis 15 | 6,0 bis 10 | 6,0 bis 10 |
| Glycerin | 40 bis 94 | 45 bis 92 | 60 bis 90 | 70 bis 88 | 70 bis 88 |
| Stearoylglutamat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

| | Formel 151 | Formel 152 | Formel 153 | Formel 154 | Formel 155 |
|---|---|---|---|---|---|
| Silica Dimethicone Silylate | 5 bis 20 | 5,5 bis 18 | 6,0 bis 15 | 6,0 bis 10 | 6,0 bis 10 |
| Sorbitol | 40 bis 94 | 45 bis 92 | 60 bis 90 | 70 bis 88 | 70 bis 88 |
| Stearoylglutamat | 0,1 bis 15 | 1,0 bis 12 | 2,0 bis 11 | 5,0 bis 10 | 5,0 bis 10 |
| Wasser | 10 bis 40 | 10 bis 25 | 10 bis 25 | 10 bis 25 | 15 bis 25 |

| | Formel 156 | Formel 157 | Formel 158 | Formel 159 | Formel 160 |
|---|---|---|---|---|---|
| Silica Dimethicone Silylate | 5 bis 20 | 5,5 bis 18 | 6,0 bis 15 | 6,0 bis 10 | 6,0 bis 10 |
| Panthenol | 40 bis 94 | 45 bis 92 | 60 bis 90 | 70 bis 88 | 70 bis 88 |
| Stearoylglutamat | 0,1 bis 15 | 1,0 bis 12 | 2,0 bis 11 | 5,0 bis 10 | 5,0 bis 10 |
| Wasser | 0 bis 40 | 0 bis 25 | 0 bis 10 | < 1,0 | -- |

1) enthält hydrophobiertes Silikat
2) enthält mindestens eine Verbindung aus der Gruppe Glycerin, Sorbitol und Panthenol
3) enthält einen bei Raumtemperatur (20°C) festen anionischen Emulgator
Die erfindungsgemäßen kosmetischen Zusammensetzungen können weitere Hilfs-, Pflege- und Zusatzstoffe enthalten. Der Gewichtsanteil der in den erfindungsgemäßen pulverförmigen Zusammensetzungen neben den Komponenten a) bis d) enthaltenen weiteren Inhaltsstoffe, insbesondere der in diesen Zusammensetzungen enthaltenen weiteren Hilfs-, Pflege- und Zusatzstoffe am Gesamtgewicht der erfindungsgemäßen pulverförmigen Zusammensetzungen beträgt vorzugsweise weniger als 10 Gew.-%, bevorzugt weniger als 5,0 Gew.-%, besonders bevorzugt weniger als 2,0 Gew.-% und insbesondere weniger als 1,0 Gew.-%. Der Gewichtsanteil dieser Hilfs-, Pflege- und Zusatzstoffe am Gesamtgewicht der erfindungsgemäßen kosmetischen Mittel kann beispielsweise 0,001 bis 2 Gew.-%, insbesondere 0,01 bis 0,5 Gew.-%, betragen.

Bevorzugte erfindungsgemäße kosmetische Mittel enthalten mindestens einen Ölkörper. Zu den natürlichen und synthetischen kosmetischen Ölkörpern zählen beispielsweise pflanzliche Öle, flüssige Paraffinöle, Isoparaffinöle und synthetische Kohlenwasserstoffe, der Silikonöle sowie Di-n-alkylether mit insgesamt zwischen 12 bis 36 C-Atomen, insbesondere 12 bis 24 C-Atomen.

Eine erste bevorzugte Gruppe von Ölkörpern sind die pflanzlichen Öle. Beispiele für solche Öle sind Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Orangenöl, Palmöl, Pfirsichkernöl, Rapsöl, Reisöl, Sanddornfruchtfleischöl, Sanddornkernöl, Sesamöl, Sheabutter, Sojaöl, Sonnenblumenöl, Traubenkernöl, Walnußöl, Weizenkeimöl, Wildrosenöl und die flüssigen Anteile des Kokosöls. Geeignet sind aber auch andere Triglyceridöle wie die flüssigen Anteile des Rindertalgs sowie synthetische Triglyceridöle.

Bevorzugte Ölkörper entstammen der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von C₆ - C₃₀ - Fettsäuren mit C₂ - C₃₀ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte Ölkörper.

In Bezug auf die kosmetische Wirkung haben sich kosmetische Zusammensetzungen als vorteilhaft erwiesen, bei denen der Gewichtsanteil des Ölkörpers am Gesamtgewicht der Zusammensetzung 0,01 bis 5,0 Gew.-%, vorzugsweise 0,02 bis 4,0 Gew.-% und insbesondere 0,05 bis 2,0 Gew.-% beträgt.

Als weiteren Pflegestoff kann das Mittel ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Als Pflegestoff können die erfindungsgemäßen Mittel weiterhin mindestens einen Pflanzenextrakt, aber auch Mono- bzw. Oligosaccharide und/oder Lipide enthalten.

Die erfindungsgemäßen pulverförmigen Zusammensetzungen können als Pflegestoff mindestens einen UV-Filter enthalten. Die UV-Filter sind in den erfindungsgemäßen pulverförmigen Zusammensetzungen üblicherweise in Mengen 0,01-5 Gew.-%, bezogen auf die erfindungsgemäße pulverförmige Zusammensetzung, enthalten. Mengen von 0,1-2,5 Gew.-% sind bevorzugt.

Die erfindungsgemäßen pulverförmigen Zusammensetzungen können in beliebigen Behältnissen konfektioniert werden, sofern sichergestellt ist, dass die mechanische Belastung des Pulvers bei der Entnahme der Zusammensetzung nicht zu einer Verflüssigung führt. Geeignet sind beispielsweise Tiegel, Flaschen oder auch Tetrapacks, wobei das Behältnis beispielsweise mit einer Schütt- und Dosiervorrichtung ausgestaltet werden kann.

Ein weiterer Gegenstand der vorliegenden Anmeldung ist die Verwendung einer erfindungsgemäßen kosmetischen Zusammensetzung zur temporären Verformung keratinhaltiger Fasern, insbesondere menschlicher Haare. Bei der Verwendung der pulverförmigen Zusammensetzung zur temporären Verformung keratinischer Fasern wird vorzugsweise zunächst die gewünschte Menge der pulverförmigen Zusammensetzung dem Behältnis entnommen. Die Zusammensetzung kann dabei direkt auf die zu behandelnde keratinische Faser oder aber beispielsweise auf die Hand gegeben werden. Im ersten Fall kann das aufgebrachte Pulver direkt auf der keratinischen Faser einer mechanischen Belastung, beispielsweise mittels der Hände, ausgesetzt werden, wodurch die flüssige, wässrige Phase direkt auf der Faser freigesetzt wird. Wird die pulverförmige Zusammensetzung zunächst auf die Hand gegeben, so kann es zunächst vorsichtig im Haar verteilt und wiederum erst anschließend stärker mechanisch belastet werden, beispielsweise durch gezieltes Einmassieren des Pulvers in das Haar. Dadurch wird die flüssige, wässrige Phase auf dem Haar freigesetzt.
Es ist natürlich auch möglich, die pulverförmige Zusammensetzung bereits auf der Hand zu verreiben und erst das entstehende flüssige oder pastenartige Mittel auf die keratinische Faser aufzubringen. Diese Vorgehensweise ist allerdings nicht bevorzugt, da dabei auf einen wesentlichen Vorteil der pulverförmigen Konsistenz des Stylingmittels, nämlich die gute Verteilbarkeit, verzichtet wird. Die pulverförmige Zusammensetzung lässt sich natürlich auch mit einem Hilfsmittel, etwa einem Pinsel, einem Schwamm, einem Tuch, einer Bürste oder einem Kamm auftragen.

Ein weiterer Gegenstand der vorliegenden Anmeldung ist ein Verfahren zur temporären Verformung keratinhaltiger Fasern, insbesondere menschlicher Haare, bei welchem die keratinischen Fasern mit einer erfindungsgemäßen kosmetischen Zusammensetzung beaufschlagt und temporär in ihrer Form fixiert werden, dadurch gekennzeichnet, dass aus der kosmetischen Zusammensetzung vor, während oder nach dem Aufbringen auf die keratinische Fasern durch Einwirkung einer Kraft eine plastisch verformbare Masse gebildet wird.

Offenbart wird schließlich auch ein Verfahren zur Herstellung erfindungsgemäßer pulverförmiger kosmetische Zusammensetzung, enthaltend
a) 5 bis 20 Gew.-% hydrophobiertes Metalloxidpulver, enthaltend hydrophobiertes Silikat,
b) 40 bis 94 Gew.-% organisches Polyol, enthaltend mindestens eine Verbindung aus der Gruppe Glycerin, Sorbitol und Panthenol,
c) 0,1 bis 15 Gew.-% Emulgator, enthaltend einen bei Raumtemperatur (20°C) festen anionischen Emulgator,
d) 0 bis 40 Gew.-% Wasser
umfassend die Schritte
i) Vermischen der Bestandteile b) und gegebenenfalls d);
ii) Einbringen des hydrophobierten Metalloxidpulvers a) in die aus Schritt i) resultierende Mischung und erneutes Vermischen, vorzugsweise für eine Zeitdauer von 1 bis 120 Sekunden, bevorzugt von 10 bis 60 Sekunden, unter Ausbildung eines Pulvers;
iii) Einbringen des vorzugsweise pulverförmigen Emulgators in das in Schritt ii) erzeugte Pulver und erneutes Vermischen, vorzugsweise für eine Zeitdauer von 1 bis 120 Sekunden, vorzugsweise von 10 bis 60 Sekunden.

### Beispiele

Es wurden wie nachfolgend beschrieben die pulverförmigen Stylingmittel V1 bis V7 hergestellt, (Angaben in Gew.-%):

| **Rohstoffe** | **V1** | V2 | V3 | **V4** | **V5** | **V6** | **V7** |
|---|---|---|---|---|---|---|---|
| Aerosil® R 202 ¹⁾ | 6,4 | 7,50 | 6,4 | 6,4 | 6,4 | 6,4 | 6,4 |
| D-Panthenol | -- | -- | -- | 84,6 | 84,6 | -- | -- |
| Glycerol | -- | 35,0 | 84,6 | | | 84,6 | |
| Neosorb 70/70 ²⁾ | 84,6 | 48,5 | | | | | 84,6 |
| Eumulgin Prisma ³⁾ | -- | -- | -- | -- | 9,0 | 9,0 | 4,0 |
| Eumulgin SG ⁴⁾ | 9,0 | 9,0 | 9,0 | 9,0 | -- | -- | 5,0 |

| | | | | | | | |
|---|---|---|---|---|---|---|---|
| 1) mit Polydimethylsiloxan nachbehandelte pyrogene Kieselsäure (INCI Bezeichung: Silica Dimethicone Silylate) 2) Sorbitol, 70% in Wasser 3) Dinatrium Cetearylsulfosuccinat 4) Natrium Stearoylglutamat | | | | | | | |

Alle Bestandteile bis auf Aerosil® R 202 und den Emulgator wurden in einem Gefäß gemischt. Diese Flüssigkeit wurde mit dem hydrophobierten Siliziumdioxidpulver Aerosil® R 202 S versetzt. Nach einer Rührzeit von jeweils 30 bis 45 Sekunden hatte sich jeweils ein stabiles Pulver gebildet. Anschließend wurde durch Rühren (etwa 15 Sekunden) in dem stabilen Pulver der Emulgator verteilt. Das so erhaltene fertige Stylingpulver wurde in Polyethylenflaschen abgefüllt.

## Patentansprüche

1. Pulverförmige kosmetische Zusammensetzung, enthaltend
a) 5 bis 20 Gew.-% hydrophobiertes Metalloxidpulver, enthaltend hydrophobiertes Silikat,
b) 40 bis 94 Gew.-% organisches Polyol, enthaltend mindestens eine Verbindung aus der Gruppe Glycerin, Sorbitol und Panthenol,
c) 0,1 bis 15 Gew.-% Emulgator, enthaltend einen bei Raumtemperatur (20°C) festen anionischen Emulgator,
d) 0 bis 40 Gew.-% Wasser.

2. Zusammensetzung nach Anspruch 1, **dadurch gekennzeichnet, dass** sie das hydrophobierte Metalloxidpulver bezogen auf ihr Gesamtgewicht in Mengen von 5,5 bis 18, vorzugsweise von 6 bis 15 Gew.-% enthält.

3. Zusammensetzung nach einem der vorherigen Ansprüche, **dadurch gekennzeichnet, dass** sie als hydrophobiertes Silikat eine mit Polydimethylsiloxan nachbehandelte pyrogene Kieselsäure enthält.

4. Zusammensetzung nach einem der vorherigen Ansprüche, **dadurch gekennzeichnet, dass** sie das organische Polyol bezogen auf ihr Gesamtgewicht in Mengen von 45 bis 92 Gew.-%, vorzugsweise von 60 bis 90 Gew.-% und insbesondere von 70 bis 88 Gew.-% enthält.

5. Zusammensetzung nach einem der vorherigen Ansprüche, **dadurch gekennzeichnet, dass** sie den Emulgator bezogen auf ihr Gesamtgewicht in Mengen von 1,0 bis 12 Gew.-%, vorzugsweise 2,0 bis 11 Gew.-% und insbesondere von 5,0 bis 10 Gew.-% enthält.

6. Zusammensetzung nach einem der vorherigen Ansprüche, **dadurch gekennzeichnet, dass** sie als bei Raumtemperatur (20°C) festen anionischen Emulgator mindestens einen Emulgator aus der Gruppe der Cetearyl Sulfosuccinate und Stearoyl Glutamate enthält.

7. Zusammensetzung nach einem der vorherigen Ansprüche, **dadurch gekennzeichnet, dass** sie bezogen auf ihr Gesamtgewicht weniger als 25 Gew.-%, vorzugsweise weniger als 10 Gew.-%, bevorzugt weniger als 5,0 Gew.-% und insbesondere weniger als 1,0 Gew.-% Wasser enthält.

8. Verwendung einer kosmetischen Zusammensetzung nach einem der vorherigen Ansprüche zur temporären Verformung keratinhaltiger Fasern, insbesondere menschlicher Haare.

9. Verfahren zur temporären Verformung keratinhaltiger Fasern, insbesondere menschlicher Haare, bei welchem die keratinischen Fasern mit einer kosmetischen Zusammensetzung nach einem der Ansprüche 1 bis 7 beaufschlagt und temporär in ihrer Form fixiert werden, **dadurch gekennzeichnet, dass** aus der kosmetischen Zusammensetzung vor, während oder nach dem Aufbringen auf die keratinische Fasern durch Einwirkung einer Kraft eine plastisch verformbare Masse gebildet wird.

## Claims

1. A powdered cosmetic composition, containing
a) 5 to 20 wt.% hydrophobized metal oxide powder containing hydrophobized silicate,
b) 40 to 94 wt.% organic polyol containing at least one compound from the group of glycerol, sorbitol and panthenol,
c) 0.1 to 15 wt.% emulsifier containing an anionic emulsifier which is solid at room temperature (20 °C),
d) 0 to 40 wt.% water.

2. The composition according to claim 1, **characterized in that** it contains the hydrophobized metal oxide powder in amounts of from 5.5 to 18, preferably from 6 to 15 wt.%, based on the total weight thereof.

3. The composition according to one of the preceding claims, **characterized in that** it contains, as hydrophobized silicate, a pyrogenic silicic acid post-treated with polydimethylsiloxane.

4. The composition according to one of the preceding claims, **characterized in that** it contains the organic polyol in amounts of from 45 to 92 wt.%, preferably from 60 to 90 wt.%, and in particular from 70 to 88 wt.%, based on the total weight thereof.

5. The composition according to one of the preceding claims, **characterized in that** it contains the emulsifier in amounts of from 1.0 to 12 wt.%, preferably from 2.0 to 11 wt.%, and in particular from 5.0 to 10 wt.%, based on the total weight thereof.

6. The composition according to one of the preceding claims, **characterized in that** it contains, as an anionic emulsifier which is solid at room temperature (20 °C), at least one emulsifier from the group of cetearyl sulfosuccinates and stearoyl glutamates.

7. The composition according to one of the preceding claims, **characterized in that** it contains less than 25 wt.%, preferably less than 10 wt.%, more preferably less than 5.0 wt.%, and in particular less than 1.0 wt.%, water, based on the total weight thereof.

8. The use of a cosmetic composition according to one of the preceding claims for temporarily reshaping keratin-containing fibers, in particular human hair.

9. A method for temporarily reshaping keratin-containing fibers, in particular human hair, in which a cosmetic composition according to one of claims 1 to 7 is applied to the keratin fibers and the shape of said fibers is temporarily retained, **characterized in that** before, during, or after application to the keratin fibers, a plastically deformable mass is formed from the cosmetic composition by the action of a force.

## Revendications

1. Composition cosmétique pulvérulente, contenant
a) 5 à 20 % en poids de poudre d'oxyde métallique hydrophobe, contenant du silicate hydrophobe,
b) 40 à 94 % en poids de polyol organique, contenant au moins un composé choisi dans le groupe constitué par la glycérine, le sorbitol et le panthénol,
c) 0,1 à 15 % en poids d'émulsifiant, contenant un émulsifiant anionique solide à température ambiante (20 °C),
d) 0 à 40 % en poids d'eau.

2. Composition selon la revendication 1, **caractérisée en ce qu'**elle contient la poudre d'oxyde métallique hydrophobe en des quantités de 5,5 à 18, de préférence de 6 à 15 % en poids par rapport à son poids total.

3. Composition selon l'une des revendications précédentes, **caractérisée en ce qu'**elle contient, comme silicate hydrophobe, un acide silicique pyrogène post-traité au polydiméthylsiloxane.

4. Composition selon l'une des revendications précédentes, **caractérisée en ce qu'**elle contient le polyol organique en des quantités de 45 à 92 % en poids, de préférence de 60 à 90 % en poids et en particulier de 70 à 88 % en poids par rapport à son poids total.

5. Composition selon l'une des revendications précédentes, **caractérisée en ce qu'**elle contient l'émulsifiant en des quantités de 1,0 à 12 % en poids, de préférence de 2,0 à 11 % en poids et en particulier de 5,0 à 10 % en poids par rapport à son poids total.

6. Composition selon l'une des revendications précédentes, **caractérisée en ce qu'**elle contient au moins un émulsifiant choisi dans le groupe constitué par les sulfosuccinates de cétéaryle et les glutamates de stéaroyle comme émulsifiant anionique solide à température ambiante (20 °C).

7. Composition selon l'une des revendications précédentes, **caractérisée en ce qu'**elle contient moins de 25 % en poids, de préférence moins de 10 % en poids, de préférence moins de 5,0 % en poids et en particulier moins de 1,0 % en poids d'eau par rapport à son poids total.

8. Utilisation d'une composition cosmétique selon l'une des revendications précédentes, destinée à la déformation temporaire de fibres kératiniques, en particulier de cheveux humains.

9. Procédé de déformation temporaire de fibres kératiniques, en particulier de cheveux humains, dans lequel les fibres kératiniques sont sollicitées par une composition cosmétique selon l'une des revendications 1 à 7 et sont fixées temporairement dans leur forme, **caractérisé en ce qu'**une masse plastiquement déformable est formée à partir de la composition cosmétique avant, pendant ou après l'application sur les fibres kératiniques sous l'effet d'une force.
